(19) Europäisches Patentamt
European Patent Office
Office européen des brevets

(11) EP 3 040 889 A1

(12) EUROPEAN PATENT APPLICATION

(43) Date of publication:
**06.07.2016 Bulletin 2016/27**

(51) Int Cl.:
***G06F 19/00*** *(2011.01)*

(21) Application number: **14200624.6**

(22) Date of filing: **30.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Danmarks Tekniske Universitet**
**2800 Kgs. Lyngby (DK)**

(72) Inventors:
- **Settnes, Mikkel**
**2860 Søborg (DK)**
- **Power, Stephen R.**
**2800 Kgs. Lyngby (DK)**
- **Petersen, Dirch Hjorth**
**2870 Dyssegård (DK)**
- **Jauho, Antti-Pekka**
**2400 København NV (DK)**

(74) Representative: **Inspicos P/S**
**Kogle Allé 2**
**2970 Hørsholm (DK)**

# (54) Method for predicting the electronic properties of a system

(57) The present invention relates to a method for predicting the electronic properties of a two-dimensional system, the method comprising the steps of considering the two-dimensional system as an infinite system without boundaries, converting the infinite system to a two-dimensional system having one or more boundaries, and applying an analytical approach to the two-dimensional system in order to predict the electronic properties of the two-dimensional system when said two-dimensional system is considered an infinite system.

EP 3 040 889 A1

## Description

## I. INTRODUCTION

Following the isolation of graphene a general class of two dimensional materials with widely diverse and unique electrical, mechanical and optical properties has been realized.[1,2] Two dimensional materials are almost entirely surface and are therefore very susceptible to external influences like direct patterning[3], adsorbate atoms[4], strain[5], *etc.* This variety of ways to alter and control the material properties opens a huge range of engineering possibilities.[6] In this context, it becomes important to investigate large scale disorder or patterning in relation to the electronic properties of graphene and related two dimensional materials. From a theoretical perspective there are a variety of methods available.[7] Typically, the electronic structure of the system is described with a tight-binding type Hamiltonian and a popular approach is then to construct the entire system in a piece-wise manner using recursive Green's functions (RGFs).[8] In this way, we extract the necessary terms for calculating physical quantities of interest. This method is best-suited for systems which are either finite or periodic in one dimension. It is frequently used for modeling transport, where self energies calculated using recursive techniques are used to attach semi-infinite pristine leads to either side of a finite device region.[9] Alternatively, an efficient approach to large disordered systems is the real space Kubo-Greenwood approach.[10] However, this method is unable to include open boundary conditions and can only obtain average system quantities, as opposed to local electronic and transport properties.

In the most common formulation, the RGF method treats (quasi) one dimensional systems with only two leads. Although variants of the method can be used for arbitrary geometries and multiple leads,[11,12] the method remains limited to finite-width or periodic systems. Consequently, it is unable to describe local and non-periodic perturbations, or point-like probes similar to those considered experimentally[13,14]. An extension of recursive techniques, to allow efficient treatment of local properties in systems without periodicity or finite sizes, would allow for easier theoretical investigation of systems which are computationally very expensive, or completely out of reach, using existing methods.

In this paper, we develop a Green's function (GF) method which is able to efficiently treat large and finite sized 'patches' embedded in an extended system, as shown in Fig. 1. The method combines an analytical formulation of the Green's functions describing a pristine system[15,15] with an adaptive recursive Green's function method to described the patches. It allows for calculation of both local electronic and transport properties and for the inclusion of multiple leads and arbitrary geometries embedded within an extended sample.

This patched Green's function method exploits an efficient calculation of the GF for an infinite pristine system using complex contour techniques. Using this GF, an open boundary self energy term can be included in the device Hamiltonian to describe its connection to an extended sample. The device region itself, containing nanostructures and/or leads, is then treated with an adaptive recursive method. We demonstrate the formulation using graphene, but it is generally applicable to all (quasi) 2D structures where the Green's function for the infinite pristine system can be determined. Consequently, the patched Green's function method will prove a versatile tool for efficient investigation of non-periodic nanostructures in extended two dimensional systems.

The rest of the paper is outlined as follows: the general formalism is developed in Section II A by calculating the open boundary self energy from the pristine GF. In Section II B we use graphene as an example to show the calculation of the pristine GF, while Section II C discusses the adaptive recursive method used to treat the device when including the boundary self energy. In Section III

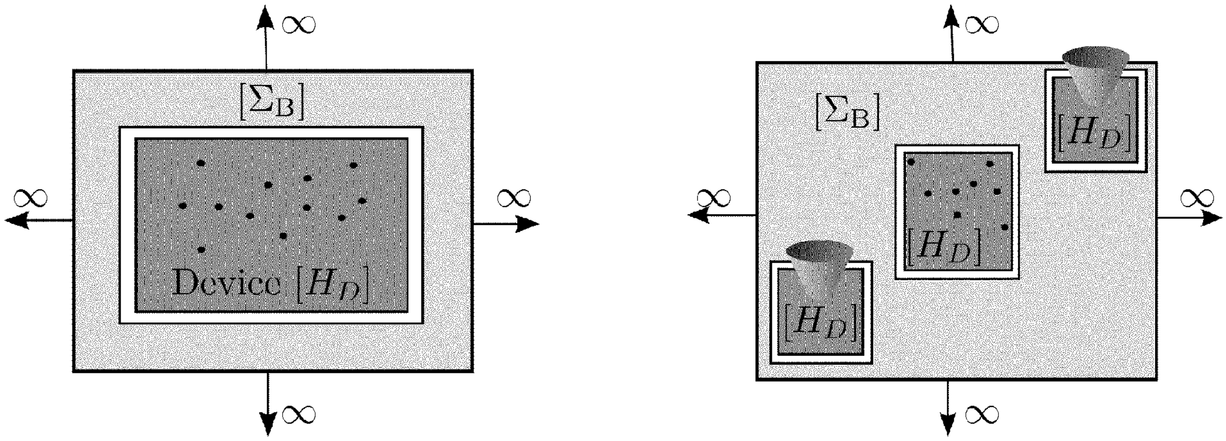


FIG. 1. The left panel shows a schematic of a computational setup containing a finite device 'patch', described by $\boldsymbol{H}_D$, embedded within an extended system described by the self energy $\boldsymbol{\Sigma}_B$. The right panel shows a computational setup containing several device 'patches' of interest.

we demonstrate how the developed method allows us to study the local density of states of a graphene sample under the influence of a local strain field. As a result, we can compare local density of state (LDOS) maps with pseudomagnetic field distributions. In this way, we show the existence of Friedel-type oscillations along with pseudomagnetic field effects in the LDOS. Finally, in Section IV, we use the patched Green's function technique to demonstrate the existence of vortex like current patterns in the presence of a perforation within an extended graphene sheet.

## II. METHOD

We consider the computational setup schematically shown in the left panel of Fig. 1, where a device region is embedded within an extended two dimensional system. The device region is described by a Hamiltonian, ***H***, which may include disorder, interaction or mean field terms. This device region is embedded into an extended system by applying a self energy term, $\Sigma_B$. Therefore we need two things: first, we need to construct $\Sigma_B$ to describe the extended part of the system and secondly, we need an efficient way to describe the device region while taking $\Sigma_B$ into account. Furthermore, the treatment of the device should be able to consider arbitrary geometries, including non-connected patches within the extended system, as shown in the right panel of Fig. 1. The method section can be outlined as follows:

A: Derivation of boundary self energy term, $\Sigma_B$, in terms of connected and disconnected pristine lattice GFs.
B: Calculation of real-space GF needed in the self energy calculation. We consider the example case of graphene.
C: Implementation of an adaptive RGF method to build the device region(s) efficiently and include the self energy term(s) $\Sigma_B$.

### A. Boundary self energy

We begin by considering the setup shown in Fig. 2a, where a device region embedded into an extended sheet is indicated by the dashed square. In this case both are assumed to be graphene-based, but the following arguments are general to any two dimensional material. We consider a division of the system into two parts: sites in the device (D) or sites in the extended sheet region. Furthermore, we subdivide the extended sheet into boundary sites (B) which have a non-zero Hamiltonian element coupling them to the device region, or 'sheet' sites which do not couple to the device region. Within a nearest-neighbour tight-binding Hamiltonian, the boundary sites in Fig. 2a are shown by blue symbols and have non-zero couplings to the device sites indicated by red symbols. We can now write the Hamiltonian for the entire system, in block matrix form, as

$$\boldsymbol{H} = \begin{pmatrix} \boldsymbol{H}_{D,D} & \boldsymbol{V}_{D,B} & 0 \\ \boldsymbol{V}_{B,D} & \boldsymbol{H}_{B,B} & \boldsymbol{V}_{B,\mathrm{sheet}} \\ 0 & \boldsymbol{V}_{\mathrm{sheet},B} & \boldsymbol{H}_{\mathrm{sheet}} \end{pmatrix}, \qquad (1)$$

where the infinite Hamiltonian surrounding the device region is indicated by the light shaded part of Eq. (6) and the

connections between device and sheet, (i.e. between the red and blue symbol sites) are contained in the off-diagonal blocks $\boldsymbol{V}_{D,B}$ and $\boldsymbol{V}_{B,D}$.

We aim to replace this infinite Hamiltonian, $\boldsymbol{H}$, with a finite effective Hamiltonian, $\boldsymbol{H}_{eff} = \boldsymbol{H}_{D,D} + \Sigma_B$, which takes into account the extended sheet using a self energy term $\Sigma_B$. To do this, we consider the *connected* system in

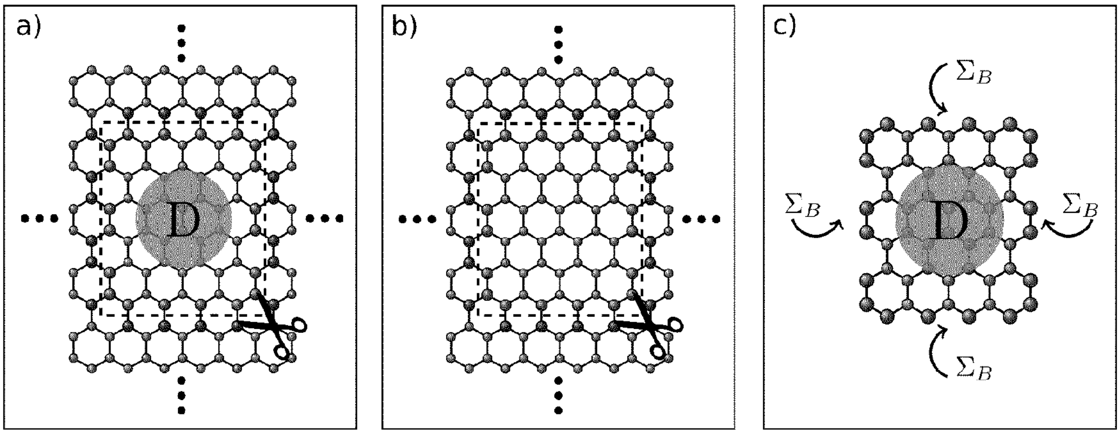


FIG. 2. a) Shows the desired device region, indicated by the dashed square, embedded within an extended system. Red symbols are the edge of the device and blue symbols indicate sites in the surrounding sheet that couples to the device. We obtain the disconnected system discussed in the main text by removing the couplings along the dashed line. b) Shows the corresponding pristine system. Again the disconnected system is obtained by removing couplings along the dashed line. c) Illustrates how the effect of the extended sheet on the device region is taken into account by the self energy, see Eq. (4).

panel a) of Fig. 2, and a disconnected system formed by removing the Hamiltonian elements $\boldsymbol{V}_{D,B}$ and $\boldsymbol{V}_{B,D}$, corresponding to removing couplings along the dashed line in Fig. 2a. The GFs of the connected ($\boldsymbol{G}^{(\mathrm{con})}$) and disconnected ($\boldsymbol{G}^{(\mathrm{dis})}$) systems can be related via the Dyson equation, and in particular we can write the GF of the connected device region as

$$\boldsymbol{G}^{(\mathrm{con})}_{D,D} = \boldsymbol{G}^{(\mathrm{dis})}_{D,D} + \boldsymbol{G}^{(\mathrm{dis})}_{D,D} \boldsymbol{V}_{D,B} \boldsymbol{G}^{(\mathrm{con})}_{B,D}. \qquad (2)$$

Applying the Dyson equation again to Eq. (2) allows us to simplify this further,

$$\boldsymbol{G}^{(\mathrm{con})}_{D,D} = \left(E\mathbf{1} - \boldsymbol{H}_{D,D} - \boldsymbol{\Sigma}_B\right)^{-1}, \qquad (3)$$

where the self energy term is given by

$$\boldsymbol{\Sigma}_B = \boldsymbol{V}_{D,B} \boldsymbol{G}^{(\mathrm{dis})}_{B,B} \boldsymbol{V}_{B,D}. \qquad (4)$$

We note that the self energy in Eq. (4) is independent of the considered device and depends only on GF matrix elements connecting sites in the pristine surrounding 'frame' that remains when the device is removed from the full system. We take advantage of this to temporarily replace our device with a corresponding pristine region of the same size, as shown in panel b) of Fig. 2. The self-energy required to incorporate the finite pristine region into an infinite, pristine sheet is the same self-energy, $\Sigma_B$, that is required in Eq. (2). We can therefore write the required GF matrix element, $\boldsymbol{G}^{(\mathrm{dis})}_{B,B}$, in terms of the GFs of the infinite pristine sheet, $\boldsymbol{G}^{(0)}$, using the Dyson equation,

$$\boldsymbol{G}^{(\mathrm{dis})}_{B,B} = \left(\mathbf{1} + \boldsymbol{G}^{(0)}_{B,D} \boldsymbol{V}_{D,B}\right)^{-1} \boldsymbol{G}^{(0)}_{B,B}. \qquad (5)$$

The advantage of writing the self-energy in terms of the pristine sheet GFs $\boldsymbol{G}_{B,B}^{(\mathrm{con})}$ and $\boldsymbol{G}_{B,D}^{(\mathrm{con})}$ becomes clear in the next section where we demonstrate an efficient method to calculate these two terms. It is worth noting that $\boldsymbol{G}_{B,D}^{(\mathrm{con})}$ only needs to be calculated for the sites in D which connect to sites in B. These sites are indicated by red in Fig. 2 and are where the self-energy terms need to be added, as shown in panel c).

The calculation scheme can be summarized as follows:

1: Calculate $\boldsymbol{G}_{B,B}^{(0)}$ and $\boldsymbol{G}_{B,D}^{(0)}$ using the methods outlined in Section II B.

2: Calculate $\Sigma_B$ from Eq. (4) and Eq. (5).

3: The finite GF for the device region, $\boldsymbol{G}_{D,D}^{(\mathrm{con})}$, is given by Eq. (3) and can be treated using an adaptive RGF method, see Section II C.

We note that this approach does not require a specific form of the device, nor does the device region need to be contiguous. We can treat different non-connected patches in an extended system, as shown in the right panel of Fig. 1, by extending the set D to include sites inside each patch and similarly expanding B to include sites at the boundary of each patch. The method presented in this section is applicable to any system where the connected, pristine GFs are easily obtainable. In practice this requires that $\boldsymbol{G}_{B,B}^{(\mathrm{con})}$ and $\boldsymbol{G}_{B,D}^{(\mathrm{con})}$ can be obtained in an analogous manner to that shown in the next section using a tight-binding description of graphene as an example.

**B. Real space graphene Green's function**

We now turn to the calculation of the real space GF of the pristine system, which is needed to calculate the self energy, $\Sigma_B$, in Eq. (4) and Eq. (5). The approach required to calculate this quantity is demonstrated below for the case of a graphene sheet described with a nearest-neighbour tight-binding Hamiltonian, but is easy generalised for other cases.

This Hamiltonian is given by,

$$\boldsymbol{H} = \sum_{<i,j>} \gamma_{cc} \hat{c}_i^{\dagger} \hat{c}_j, \qquad (6)$$

where the sum $< i, j >$ runs over all nearest neighbour pairs and the carbon-carbon hopping integral is $\gamma_{cc} \approx -2.7$ eV. The graphene hexagonal lattice can be split into two triangular sublattices, which we denote A and B, and neighbouring sites reside on opposite sublattices to each other. Using Bloch functions, the Hamiltonian can be rewritten in reciprocal space as[7]

$$\boldsymbol{H}_{\boldsymbol{k}} = \gamma_{cc} \begin{pmatrix} 0 & f(\boldsymbol{k}) \\ f^*(\boldsymbol{k}) & 0 \end{pmatrix}, \qquad (7)$$

where the matrix form arises from sublattice indexing within a 2 atom unit cell and we have used the definition $f(\boldsymbol{k}) = 1 + e^{i\boldsymbol{k}\cdot\boldsymbol{a}_1} + e^{i\boldsymbol{k}\cdot\boldsymbol{a}_2}$, with the lattice vectors $\boldsymbol{a}_1 = a_0(\sqrt{3},3)/2$ and $\boldsymbol{a}_2 = a_0(-\sqrt{3},3)/2$ and $a_0$ the carbon-carbon distance. With this definition of the unit vectors we have the armchair direction along the y-axis (and zigzag along the x-axis).

The eigenenergies and eigenstates of the system are easily obtained from this form of the Hamiltonian, and transforming back to real space allows us to write the desired Green's function between sites $i$ and $j$ as[16,17]

$$G_{ij}^0(z) = \frac{1}{\Omega_{BZ}} \int \mathrm{d}^2\boldsymbol{k} \frac{N_{ij}(z,\boldsymbol{k}) \mathrm{e}^{i\boldsymbol{k}\cdot(\boldsymbol{r}_j - \boldsymbol{r}_i)}}{z^2 - \gamma_{cc}^2 |f(\boldsymbol{k})|^2}, \qquad (8)$$

where $z = E + i0^+$ is the energy, $\Omega_{BZ}$ is the area of the first Brillouin zone. The position of the unit cell containing site i is denoted by $\boldsymbol{r}_i = m_i\boldsymbol{a}_1 + n_i\boldsymbol{a}_2$ with $m_i$ and $n_i$ being integers. Finally we use the definition $N_{ij}(z, \boldsymbol{k}) = z$, when $i$ and $j$ are on the same sublattice and $N_{ij}(z, \boldsymbol{k}) = \gamma_{cc}f(\boldsymbol{k})$ if $i$ is on the A sublattice and $j$ is on the B sublattice and $N_{ij}(z, \boldsymbol{k}) = \gamma_{cc}f^*(\boldsymbol{k})$ when $i$ is on B and $j$ on A.

To ease notation we introduce the dimensionless k-vectors $k_A = 3k_y a_0/2$ and $k_Z = \sqrt{3}k_x a_0/2$ such that $f(k_A, k_Z) = 1 + 2\cos(k_Z)e^{ik_A}$, and write the separation vector in terms of the lattice vectors $\boldsymbol{r}_j - \boldsymbol{r}_i = m\boldsymbol{a}_1 + n\boldsymbol{a}_2$. Inserting this into Eq. (8) gives

$$G^0_{ij}(z) = \frac{1}{2\pi^2}\int \mathrm{d}k_A \int \mathrm{d}k_Z N_{ij}(z, k_A, k_Z) \times \frac{\mathrm{e}^{\mathrm{i}k_A(m+n)+\mathrm{i}k_Z(m-n)}}{z^2 - \gamma^2_{cc}\big(1 + 4\cos^2(k_Z) + 4\cos(k_A)\cos(k_Z)\big)}. \quad (9)$$

Eq. (9) can be solved using a two-dimensional numerical integration, but as we require Eq. (9) for each Green's function element individually, we wish to increase the performance by doing one integration analytically using complex contour techniques.

Following the approach of Ref. 16, we use $k_A$ as complex variable and consider the poles, $q$, of the denominator

$$q = \cos^{-1}\left[\frac{\frac{z^2}{\gamma^2_{cc}} - 1 - 4\cos^2(k_Z)}{4\cos(k_Z)}\right]. \quad (10)$$

The sign of the pole must be selected carefully to ensure that it lies within the integration contour, *i.e.* $\mathrm{Im}(q) > 0$, for contours in the positive half plane corresponding to the situation $m + n \geq 0$. Care must also be taken with the additional phase terms that arise for opposite sublattice GFs.

Using the residue theorem and integrating over a rectangular Brillouin zone, $k_A \in [-\pi; \pi]$ and $k_Z \in [-\pi/2; \pi/2]$, we finally reduce Eq. (9) to

$$G^0_{ij} = \frac{\mathrm{i}}{4\pi\gamma^2_{cc}}\int_{-\pi/2}^{\pi/2} \mathrm{d}k_Z \frac{N_{ij}(z, q, k_Z)\mathrm{e}^{\mathrm{i}q(m+n)+\mathrm{i}k_Z(m-n)}}{\cos(k_Z)\sin(q)}, \quad (11)$$

with q given by Eq. (10). A similar expression to Eq. (11) can be derived when using $k_Z$ as first integration variable.[16] The above derivation is done using a nearest neighbour model, but can be generalised to also include, for example, second nearest neighbour terms[18] or uniaxial strains.[19]

We can now use Eq. (11) to calculate the elements of the required GFs, $\boldsymbol{G}^{(0)}_{B,B}$ and $\boldsymbol{G}^{(0)}_{B,D}$, defined in Section II A. In this way, Eq. (11) can be used to fill up the elements of the desired matrices one at a time. Since we need GF matrices of size $N_B \times N_B$ and $N_B \times N_D$, where $N_D$ and $N_B$ are the number of sites at the edge of the device region and in the region B, respectively, it could seem very ineffective to calculate one element at a time. However, the total number of GF elements to be calculated is greatly reduced by the symmetries of the pristine graphene lattice. The lattice itself is six-fold symmetric and each of these six identical wedges is in turn mirror symmetric, resulting in a 12-fold degeneracy of the GFs indexed by site separation vectors. Additionally, many of the required elements in $\boldsymbol{G}^{(0)}_{B,B}$ and $\boldsymbol{G}^{(0)}_{B,D}$ are identical. For instance, the onsite and nearest neighbour GF element appear many times, but only need to be calculated once. Taking the device region in Fig. 2 as example we have $N_D = N_B = 20$, yielding 400 individual elements for a brute force calculation. Instead, using symmetries and duplicates, we only need to calculate 38 and 42 elements when determining $\boldsymbol{G}^{(\mathrm{con})}_{B,B}$ and $\boldsymbol{G}^{(\mathrm{con})}_{B,D}$, respectively. The reduction becomes more significant for larger systems, as we generally only need to add the GF elements corresponding to the longest couplings. Consequently, only

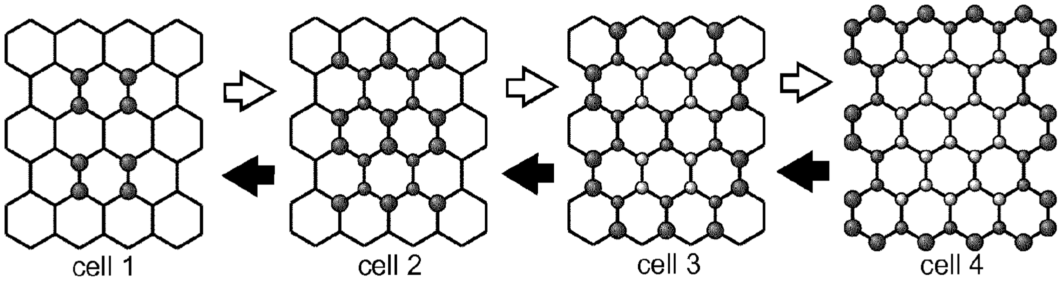


FIG. 3. The partitioning of a small graphene sample where all sites of interest are located in cell 1. Cell 2 is all the sites coupling to cell 1 but are not themselves part of cell 1. Likewise cell 3 is the sites coupling to cell 2 and so on. The red sites are assigned to the current cell and the lines indicate the sites still to be assigned. The previous cell and all sites already added are indicated by gray and white, respectively. The recursive sweep starting at the last cell ending in cell 1 (indicated by filled arrows) gives GFs connecting all sites of interest. As indicated by the empty arrows we also employ a backward recursion to obtain local properties everywhere within the device region.

a small percentage of the GF elements need to be calculated individually and their values for frequently used separations and energies can be stored or reused to enable extremely fast calculation of the required self energies.

## C. Adaptive recursion for device region

In this section we consider the device region where the boundary self energy can be added at the edge. The full GF of the device region is given by $\boldsymbol{G}_D = (E\mathbf{1} - \boldsymbol{H}_D - \Sigma_B)^{-1}$, where we have simplified the notation from Eq. (3). From this GF both transport and local properties can be obtained. However, for most purposes we do not require every element of the Green's function matrix element in the device region, and so to avoid atime consuming full matrix inversion, recursive methods are often applied[8,20-26].

This section outlines an adaptive recursion method which efficiently includes the boundary self energy as well as an arbitrary device region shape and configuration (and number) of leads. Alternative approaches have been developed to treat arbitrary shaped regions with multiple leads[11,12,26]. These so-called knitting-algorithms add single sites at a time. They rely on a complicated categorizing of sites into different intermediate updating blocks making the theory and implementation cumbersome. Hence, we use an approach similar to the ones in Refs. 20-22, and employ an adaptive partitioning of the Hamiltonian matrix in order to bring it into the desired tridiagonal form suitable for recursive methods.

Calculating physical properties generally requires certain GFs connecting a specific set of sites in the device region. These sites of interest, for example, could be sites where we want to introduce defects, or couple to probes for transport calculations, or measure properties like the local density of states. We focus first on the general partitioning process, and then demonstrate how it can be quickly modified to account for the edge self-energy terms. We begin by placing all these sites of interest into recursive cell 1, as shown by the red sites in Fig. 3. We emphasize that the cells in this process are not of a fixed size and may consist of arbitrary sites which are not necessarily connected. Cell 2 is determined by selecting all the remaining unpartitioned sites which couple directly to sites in cell 1 via a non-zero Hamiltonian matrix element. In the example in Fig. 3, this consists of nearest neighbor sites of those in cell 1, which are not themselves in cell 1. This process is repeated until all sites in the device region have been allocated, and is demonstrated schematically in the panels of Fig. 3 where red sites indicate the current cell, and dark gray or white sites indicate sites added to the previous cell, or to earlier cells, respectively.

With the resultant block tridiagonal Hamiltonian, we can now employ the usual recursive algorithm, starting from cell $n = N$, so that the final step yields the required GF sites in cell $n = 1$. These terms can then be used to calculate observable quantities like transmission, LDOS *etc.* Afterwards a reverse recursive sweep from $n = 1$ to $n = N$ can be implemented to efficiently map local quantities like bond currents or LDOS everywhere within the device region[8]. For completeness the full recursive method is summarized in Appendix A including the reverse sweep. We emphasize that the presented method is not unique to graphene systems, but can be employed to arbitrary tight-binding-like models.

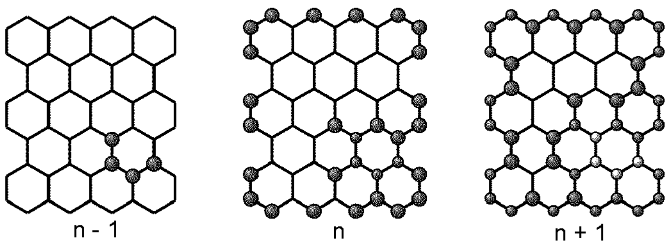


FIG. 4. An example of the partitioning when the cell $n-1$ is connected to the edge, and we need to include the boundary self-energy, $\mathbf{\Sigma}_B$. The symbols are similar to Fig. 3.

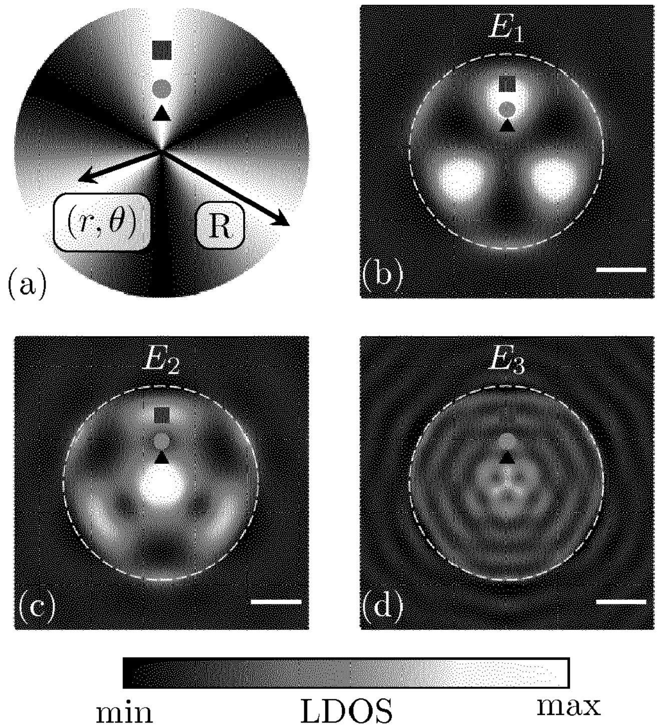


FIG. 5. (a) The PMF distribution calculated using the strain distribution in Eq. (13), dark being negative field and light being positive. (b-d) Real space LDOS maps for the sublattice A taken at the energies $E_1 = 0.06|t|$, $E_2 = 0.089|t|$ and $E_3 = 0.23|t|$, corresponding to energies of the first two pseudo Landau levels and an energy dominated by Friedel type oscillations, respectively. The energies and the symbols correspond the ones used in Fig. 6. Sublattice B is similar and is obtained by rotating 60°. The scale bar is 5 nm.

*Including the boundary self-energy*

We now return to the specific case at hand where the recursive method outlined above needs to be adapted carefully to take account of the boundary self energy. In general $\Sigma_B$ is a non-hermitian dense matrix connecting all edge sites of the device region. Therefore it is essential to assign all edge site to the same cell. This principle is shown in Fig. 4. If cell *n* - 1 contains sites which connect to at least one edge, then cell *n* must contain not only the edge sites directly connecting to cell *n* - 1, but also all other edge sites, as these are connected to each other via $\Sigma_B$. In this way, the cell, *n* + 1, must then contain all the sites connecting to cell *n, i.e.* also connecting to the edge, but not included in cell *n.* The full cell partitioning algorithm, including this step, is given in Appendix A.

## III. INHOMOGENEOUS STRAIN FIELDS IN GRAPHENE BUBBLES

In this section, we employ the patched Green's function method to a locally strained graphene system, demonstrating how it can prove a useful tool in investigating local properties of non-periodic nanostructures in extended two dimensional systems.

Strain engineering has been proposed as a method to manipulate the electronic, optical and magnetic properties of graphene.[19,27-40] It is based on the close relation between the structural and electronic properties of graphene. The application of strain can lead to effects like bandgap formation[41], transport gaps[27] and pseudomagnetic fields (PMFs).[28-30]

Uniaxial or isotropic strain will not produce PMFs, although it has been shown to shift the Dirac cone of graphene and induce additional features in the Raman signal.[42] On the other hand, inhomogeneous strain fields can introduce PMFs. In this case, the altered tight binding hoppings mimics the role of a gauge field in the low energy effective Dirac model of graphene.[43,44] For example Guinea *et al.* demonstrated that nearly homogeneous PMFs can be generated by applying triaxial strain. One of the most striking consequences of homogeneous PMFs is the appearance of a Landau-like quantization.[28,35] Scanning tunneling spectroscopy on bubble-like corrugations observe this quantization, where the observed pseudo-Landau levels corresponds to PMFs stronger than 300 T.[32,45]

Deformations can be induced in graphene samples by different techniques like pressurizing suspended graphene[30,46] or by exploiting the thermal expansion coefficients of different substrates.[32] As a result, introducing nonuniform strain distributions at the nanoscale is a promising way of realizing strain engineering. The standard theoretical approach to treat strain effects employs continuum mechanics to obtain the strain field. Several studies improve the accuracy by replacing the continuum mechanics by classical molecular dynamics simulations.[5,30,31] The strain field can then be coupled to an effective Dirac model of graphene to study the generation of PMFs in various geometries. In most studies, only the PMF distribution is considered as opposed to experimentally observable quantities like local density of states. The framework presented in Section II enables us to treat the effect of strain on the LDOS directly from a tight-binding Hamiltonian. Consequently, we are now able to describe a single bubble in an extended system without applying periodic boundary conditions which may introduce interactions between neighboring bubbles. The dual recursive sweep then allows for efficient calculation of local properties everywhere in the device region surrounding a bubble, evidently enabling us to investigate spatial variations in real space LDOS maps. In this section we only treat one nanostructure, but the patched Green's function technique efficiently handles several spatially separated nanostructures, as the separation is added very efficiently through the self energy term.

To account for strain within a tight binding approach we modify the hopping parameters.[33,37,40] The nearest neighbour hopping in Eq. (6) between site i and j is given by the new distance, $d_{ij}$, between the sites,

$$\gamma_{ij} = \gamma_{cc} \mathrm{e}^{-\beta\left(\frac{d_{ij}}{a_0}-1\right)}, \qquad (12)$$

where the coefficient $\beta = -\partial \ln t/\partial \ln a_0 \approx 3.37$.[37] We treat the deformation problem by applying an analytical displacement profile ($u(x, y)$, $z(x, y)$) matched against experimental data for pressurized suspended graphene.[47] Here $u(x, y)$ and $z(x, y)$ are the in-plane and vertical displacements, respectively, which are induced by the applied strain. For a rotational symmetric aperture with radius R, these are given, in spherical coordinates ($r$, $\theta$), as

$$z(r,\theta) = h_0\left(1 - \frac{r^2}{R^2}\right), \qquad (13a)$$

$$u(r,\theta) = u_0\frac{r}{R}\left(1 - \frac{r}{R}\right), \qquad (13b)$$

for $r < R$. Here $h_0$ is the maximal height of the bubble and $u_0 = 1.136 h_0^2/R$ is a constant relating the out-of-plane and in-plane deformation.[47] We note that this profile gives rise to a sharp edge at $r = R$, and many of the features we discuss below emerge from the strongly clamped nature of this bubble type.

As shown in Appendix B, rotationally symmetric strain profiles give rise to threefold symmetric PMFs in the effective Dirac model. This is shown in Fig. 5a for the strain profile considered in Eq. (13). As discussed in earlier studies,[33,40] we get an asymmetric sublattice occupancy such that the LDOS of each sublattice has a threefold symmetric distribution following the PMF while rotated 60° compared to the opposite sublattice. In all calculations below, we therefore

show only one sublattice, as the result for the opposite sublattice can be obtained by a 60° rotation and the total pattern is a superposition of both.[29,34]

Comparing the PMF distribution in Fig. 5a with the calculated LDOS maps at different energies in Fig. 5b-d for a bubble of radius $R$ = 10 nm and height $h_0$ = 3 nm, we immediately notice that the threefold symmetry is also present in the LDOS maps. However, the spatial LDOS maps have significant additional details compared to the PMF distribution.

In Fig. 6 we calculate the energy dependent LDOS at the positions indicated by symbols (square, circle and triangle) in Fig. 5. We first consider the average of the LDOS within the "slice" containing the symbols, shown by the bottom (red) curve in Fig. 6. Two distinct oscillation types are observed, and we argue that these can be divided into Friedel-type and PMF-induced features. At high energies in particular we notice regularly space oscillations with an approximate period of $\hbar \upsilon_F \pi / 2R$. These are consistent with Friedel-type oscillations related to the size of the structure and emerging from interferences between electrons scattered at opposite sides of the bubble. An exact treatment needs to take into account the renormalized Fermi velocity, $\upsilon_F$, due to the average change

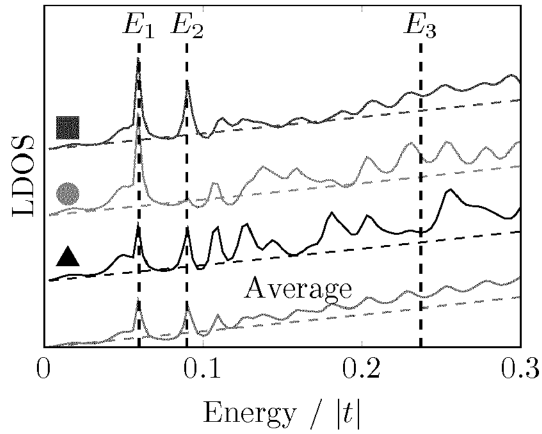


FIG. 6. The LDOS as a function of energy for the three positions indicated in Fig. 5 and for the average of the "slice" of the bubble region containing the symbols. The dashed lines indicate the LDOS without the bubble. The curves are shifted with respect to each other to increase visibility.

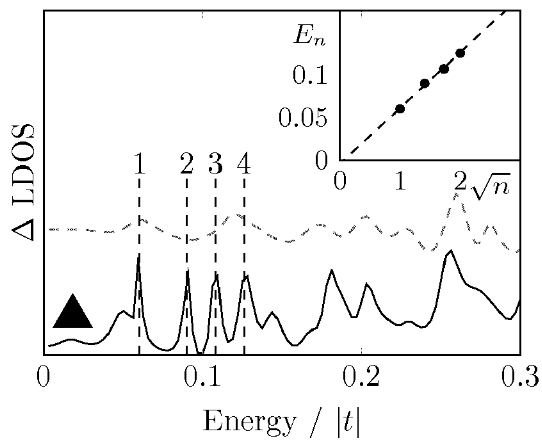


FIG. 7. The difference in LDOS as a function of energy for the point indicated with a triangle on Fig. 5. We show both the full calculation (full line) and an artificial system containing only the perturbation for a small region at the edge of the bubble (dashed line). We adjust the average hopping constant in the calculation of the artificial system to match the full calculation. Inset: The peak energies 1-4 as a function of $\sqrt{n}$, where $n$ is the peak number.

in bond length.[48] At lower energies we observe distinct peaks which are not equally spaced (the first two appear at $E_1$ and $E_2$). We will show that these are due to pseudomagnetic effects and we refer to them as pseudo Landau levels.

Besides the Friedel oscillation associated with the bubble radius, we also have similar oscillations associated with the distances to different edges of the bubble. These features are highly position dependent, and explain the differences between the three single position curves in Fig. 6. When considering the average, these position dependent oscillations are washed out (bottom curve in Fig. 6), leaving only the oscillation dependent on the structure size. However, at individual positions these oscillations can have a considerable impact. Returning to the individual position STS curves in Fig. 6, we note that the peak at $E_2$ is only dominant for the points indicated by the square and triangle. It is suppressed by Friedel-type interferences at the circle point, which is also clear from the LDOS map in Fig. 5c.

The amplitude of the Friedel-type oscillations is determined by the strength of scattering near the bubble edges. The clamped edge implied by the strength profile in Eq. (13) gives rise to significant strain fields along this edge, leading to a sharp, strong perturbation. More realistic profiles calculated from molecular dynamics also indicate strong perturbations near the edges of clamped bubbles.[30] Our results indicate that edge scattering effects may significantly effect LDOS behavior in clamped bubble systems and even mask PMF-induced features.

To treat the oscillations due to the feature size and edge sharpness in more detail, we calculate the LDOS for an artificial system only taking into account the strain field along a small ring around the edge, see Fig. 7 (dashed red line). In this way, only Friedel-type features are expected within the structure. If we compare to the full calculation (full black line in Fig. 7), we notice that the oscillations at higher energies are present in both calculations, whereas the sharp peaks are only present in the full calculation. This confirms the Friedel nature of the higher energy oscillations and suggests the lower energy peaks are due to an alternative mechanism. To confirm that the sharp peaks are due to pseudomagnetic effects, we compare the peak positions to the standard form expected for Landau levels in graphene $E_n = \mathrm{sign}(n)\sqrt{2e_0\hbar v_F^2 B_s n},$ where $e_0$ is the electron charge, $B_s$ is the magnetic field and $n$ is the peak number.[7] The peaks labelled 1-4 in Fig. 7 display the $\sqrt{n}$ dependence characteristic of Landau levels in graphene, as shown in the inset of Fig. 7. The size of the PMF can furthermore be inferred to be $B_s$ ~ 30 T from the inset.

To conclude we discussed how the features in the LDOS spectra of clamped graphene bubbles can be explained by a combination of size-dependent scattering and PMF-induced effects like pseudo Landau quantization. Significant strain fields near the edge of the structure give rise to strong Friedel-type oscillations in the LDOS and these oscillations envelope the effect of a PMF. We must therefore be careful to distinguish between the two type of oscillations when investigating the electronic effects of PMFs induced by inhomogeneous strain fields.

## IV. VORTEX CURRENTS NEAR PERFORATIONS

In this section we investigate local transport properties near antidots (i.e. perforations) in a graphene sheet. Periodic arrays of antidots have been studied as a way to open a bandgap in graphene[49-51] or to obtain waveguiding effects.[52,53] Furthermore, a single perforation in

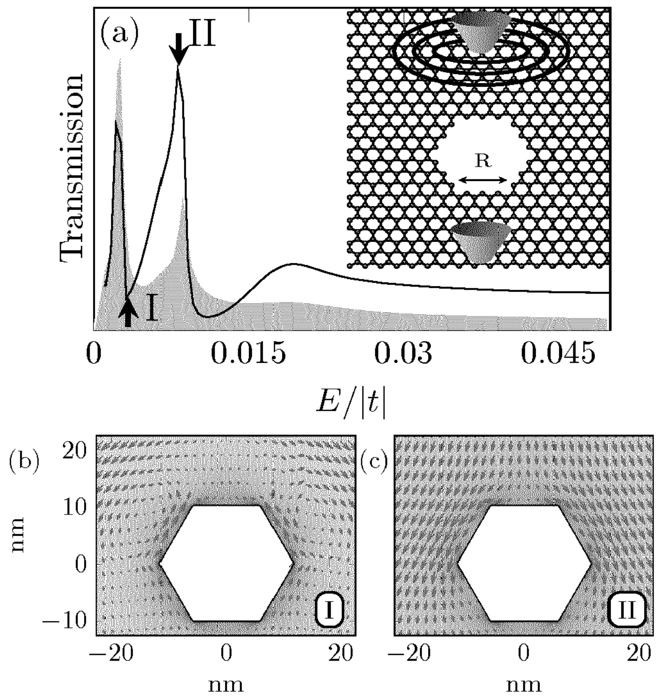


FIG. 8. (a) The transmission as a function of energy for a dual probe setup with an antidot in between the probes as schematically shown in the inset. The distance between the probes are 200 nm and the antidot with purely zigzag edges has side length $R = 48a_0 \sim 6.8$ nm. The shaded area corresponds to the LDOS around the edge of the antidot. antidot

a graphene sheet has been considered as a nanopore for DNA sensing.[54,55]

Several studies show that the electronic structure of antidots is closely related to the exact edge geometry.[49,53,56] Experimental fabrication techniques like block copolymer[3,57,58] or electron beam lithography, [59-61] inevitably lead to disorder and imperfect edges. However, it may be possible to control the edge geometry of the antidot by heat treatment[60,62], or selective etching.[61,63]

Motivated by the interest in how current flows in antidot systems, we apply the patched GF method to a single perforation in a graphene sheet. The method allows us to study the perforation with no influence from periodic repetition or finite sample size. Additionally, the combination of recursive methods and a boundary self-energy allows for investigation of antidot sizes realizable experimentally.[3,55,64] In fact we consider both an example antidot with perfect edges and an exact structure found from high resolution transmission electron microscope (TEM) images using pattern recognition.[65,66]

To investigate current on the nanoscale, recent experiments have realized multiple STM-systems.[13,14,67,68] These allow for individual manipulation of several STM-tips in order to make electrical contact to a sample in proximity of the considered nanostructure. Theoretically, we previously considered multiple STM setups allowing for both fixed and scanning probes.[56,69] The method presented here allows for not only transmission calculations but also calculation of local electronic and transport properties in the presence of multiple point probes. At the same time large separations between the different probes and/or nanostructures are easily included as additional separation is achieved in a very computationally efficient manner through the self energy term connecting multiple patches. The combination of large spatial separation between features, while still enabling calculation of local electronic and transport properties, can prove a useful tool in investigating extended two dimensional systems where we take special interest in a particular region of the extended sample.

In order to consider transmissions and current patterns, we add leads to the system through inclusion of a simple lead self-energy term, $\Sigma^L_{ij} = V^L_i g^s_{ij} V^L_{jj}$, where $V^L_i$ is the coupling element between the device site *i* the lead. We consider the lead as a continuum such that each device site only couple to the lead site directly above it, why $V^L$ is a diagonal matrix with the different couplings in the diagonal. To model the structureless lead GF, $g^s_{nm}$, we use the

surface GF of a single atomic chain, as this has a constant DOS in the considered energy range. This ensures that we do not introduce lead specific correlations into the transmission spectra. The lead needs to couple to the device such that an electron can leave the device though one site and reenter at another. In order to avoid an unphysical coupling between different lattice sites via the lead, we furthermore add a 1/r decay[70] to the off-diagonal self-energy terms as appropriate for a structureless three-dimensional free electron gas.[71]

First, we consider a zigzag-edged antidot with side length $R$ = 48a ~ 12 nm, where a is the length of the graphene unit cell and $a = \sqrt{3}a_0 = 2.46\mathring{A}$. This is comparable to experimental sizes where sub-20-nm feature sizes have been reported.[3,57?,58] The antidot is between two probes placed 200 nm apart, as shown schematically in the inset of Fig. 8a. The main panel ofFig. 8a shows the transmission as a function of energy for this dual point probe setup. We note the distinct transmission peaks. As explained in Ref. 56, these peaks are related to localized states along the zigzag edges. As a consequence, we notice the correspondence between the peaks in the transmission and the peaks in the LDOS around the edge, see shaded area on Fig. 8a.

Next, we calculate the bond currents from the top lead. The bond current between site *i* and *j* from lead L are calculated, as explained in Appendix A, by $J_{ij}^{L} = -H_{ij}\mathrm{Im}\left[\boldsymbol{G}^{a}\boldsymbol{\Gamma}^{L}\boldsymbol{G}^{r}\right]_{ij}/\hbar,$ where $H_{ij}$ is the Hamiltonian matrix element connecting site *i* and *j*. The bond currents around the zigzag antidot for the energies indicated in Fig. 8a are shown in Figs. 8b and 8c. In this way, we see that the transmission dips are related to vortex like current paths. These vortex paths create a larger "effective size" for the antidot at this energy, characterized by a region around the antidot avoided by the

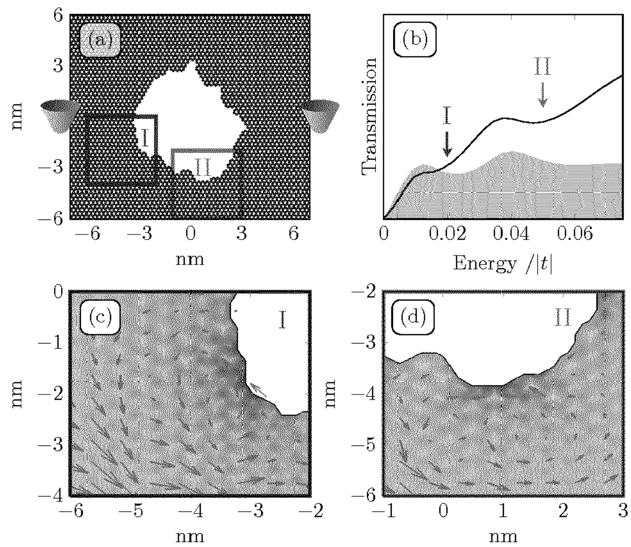


FIG. 9. An actual perforation is obtained from high resolution TEM images through pattern recognition and we consider the vortex like current paths forming around the perforation as certain energies. (a) Shows the structure of the perforation as well as an indication of the probe position (in the actual calculations the probes are 200 nm apart). The indicated areas corresponds to the zooms in (c) and (d). (b) The transmission for the dual probe setup. The shaded area indicate the average LDOS around the edge of the antidot. Furthermore, the energies I and II corresponding to the energies used on (c) and (d), respectively. (c-d) Bond current maps taken at the energies I and II, respectively, and shown at the positions indicated on (a).

current paths. On the other hand, at the transmission peaks the current passes near to the antidot edge.

The antidot considered in Fig. 8, although of realistic size, is an idealization, as experimental perforations will inevitably contain imperfections. To consider a more realistic case, we turn to a perforation observed in experimental TEM images. Using pattern recognition[65,65] the positions of the individual carbon atoms are obtained from high resolution TEM images (see Fig. 9 of Ref.[66]). Pristine graphene is added around the experimentally obtained perforation to obtain

the system shown in Fig. 9a. From the transmission (see Fig. 9b), we notice that peaks are still present, but broadened by the disorder. Considering the two energies I and II in Fig. 9b and comparing their spatial current maps, we find that certain positions around the antidot are responsible for the additional backscattering causing the transmission dips. Dip I corresponds to a vortex pattern ot the left side of the antidot (see Fig. 9c), whereas the dip at II is caused by a vortex pattern on the right side of the antidot (see Fig. 9d). This result suggests that electrons at different energies see a different effective perforation size and shape and are scattered accordingly.

V. CONCLUSION

We have expanded the standard recursive Green's function method to calculate local and transport properties to enable calculations in extended non-periodic systems. We exploit an efficient calculation of the pristine two-dimensional GF using complex contour methods. Once calculated, the pristine GFs are used to determine a boundary self energy term describing the extended system. In this way, we can treat a finite device region embedded within an extended sample.

We first demonstrated how this approach is able to efficiently treat the electronic properties of strained bubbles in an extended graphene sheet. Considering a clamped bubble, we have shown that the finite size gives rise to Friedel-type oscillations in the density of states. This effect mixes with any pseudomagnetic effects arising from the strain field. The effect of the edge for constricted geometries was observed in Ref. 30 in relation to the pseudomagnetic field distribution. Here we furthermore show that the edge effects can cloud pseudomagnetic signatures in the LDOS by adding additional structure which are not directly related to pseudomagnetic effects.

Secondly, we showed how finite leads can be added to a patched device region to efficiently calculate transport properties for spatially separated features, while still being able to map local properties in various parts of the system. In particular, we investigated the current flow around perforations of a graphene lattice. Both idealized geometries and experimental geometries obtained from high resolution TEM images were considered. The transmissions show distinct dips caused by localized states along zigzag segments of the perforations. The transmission dips could be associate with vortex-like current paths formed near the perforation edges.

We have demonstrated the versatility of this novel approach to the popular recursive GF method. The method allows for calculation of the same local and transport properties as the standard method, but adds the ability to treat large non-periodic structures embedded in extended samples. We predict that this will prove an invaluable tool in the investigation of nanostructures in two dimensional materials.

**Acknowledgements** We thank B.K. Nikolic for enlightening discussions of recursive methods applied to arbitrary geometries. We also thank J. Kling for providing the experimental TEM data used in modelling current paths around realistic perforations. The Center for Nanostructured Graphene (CNG) is sponsored by the Danish Research Foundation, Project DNRF58.

**Appendix A: Recursive Algorithm**

To obtain a tridiagonal Hamiltonian we let cell $n$ = 1 contain all sites of interest. Then following the algorithm outlined below we assign all sites into cells.

1: Let {n} denote all sites in cell *n.*
Find all sites j for which $V_{ij} \neq 0$ and call that set {*j*}.

1a: If {j} contains an edge site, all edge sites are added to the set {*j*}.

2: Sites in {*j*} not present in {"*added*"} are assigned to cell $n$ + 1.

3: Sites in {$n$ + 1} are added to {"added"}

4: Repeat 1-3 until all sites are assigned to a cell.

Step 1a is added if we add an edge self energy term $\Sigma_B$ as described in Section II.

Assuming the tridiagonal partitioning obtained from the algorithm above, we make an update sweep starting from cell $n = N,$ see schematic representation of Fig. 10. The steps are calculated using the recursive relations[8]

$$\boldsymbol{g}_{N,N} = \left(E - \boldsymbol{H}_{N,N}\right)^{-1}, \qquad \text{(A1a)}$$

$$\boldsymbol{g}_{n,n} = \big(E - \boldsymbol{H}_{n,n} - \boldsymbol{V}_{n,n+1}\boldsymbol{g}_{n+1,n+1}\boldsymbol{V}_{n+1,n}\big)^{-1}, \tag{A1b}$$

$$\boldsymbol{g}_{1,1} = \big(E - \boldsymbol{H}_{1,1} - \boldsymbol{V}_{1,2}\boldsymbol{g}_{2,2}\boldsymbol{V}_{2,1} - \sum_{m=1}^{M} \Sigma_{lead}^{m}\big)^{-1}. \tag{A1c}$$

After the sweep is complete, the full GF of cell $n$ = 1 is obtained as $G_{1,1} = \boldsymbol{g}_{1,1}$. As all sites of interest are placed in this cell, we can now calculate observables involving these sites. For example we calculate transmission, $\mathcal{T}_{L,L'}$, between lead L and L' using these GFs.

$$\mathcal{T}_{L,L'}(E) = \mathrm{Tr}\big[\boldsymbol{G}_{L',L}\boldsymbol{\Gamma}_{L,L}^{L}\boldsymbol{G}_{L,L'}^{\dagger}\boldsymbol{\Gamma}_{L',L'}^{L'}\big], \tag{A2}$$

where $\Gamma^L = i(\Sigma^L - \Sigma^{L\dagger})$.

In order to obtain other blocks of the full GF matrix, we need to store the GF matrix for each cell as we sweep from $n = N$ to $n$ = 1. The stored blocks are shown in light gay on Fig. 10.

To obtain the LDOS at site $i$, $\rho_{ii} = -\mathrm{Im}(G_{ii})/\pi$, we need the diagonal of the GF matrix. We calculate the block diagonal from a reversed sweep from $n$ = 1 to $n = N$, see Fig. 10. The reversed sweep uses the block diagonals, $\boldsymbol{g}_{n,n}$, from the first sweep to calculate the full diagonal GF, $\boldsymbol{G}$,

$$\boldsymbol{G}_{n,n} = \boldsymbol{g}_{n,n} + \boldsymbol{g}_{n,n}\boldsymbol{V}_{n,n-1}\boldsymbol{G}_{n-1,n-1}\boldsymbol{V}_{n-1,n}\boldsymbol{g}_{n,n}. \tag{A3}$$

At last we want to obtain bond currents for the state leaving a lead $L$. This can be calculated by $J_{ij}^{L} = -H_{ij}\mathrm{Im}\big[\boldsymbol{G}_{i,1}\boldsymbol{\Gamma}_{1,1}^{L}\boldsymbol{G}_{1,j}^{\dagger}\big]/\hbar$. Remembering that the leads are assigned to cell $n$ = 1, we need the off-diagonal blocks, $\boldsymbol{G}_{1,n}$ and $\boldsymbol{G}_{n,1}$, in order to obtain bond currents. Using the stored GFs from the first sweep we can calculate the needed off-diagonals,

$$\boldsymbol{G}_{1,n} = \boldsymbol{G}_{1,n-1}\boldsymbol{V}_{n-1,n}\boldsymbol{g}_{n,n}, \tag{A4a}$$

$$\boldsymbol{G}_{n,1} = \boldsymbol{g}_{n,n}\boldsymbol{V}_{n,n-1}\boldsymbol{G}_{n-1,n}. \tag{A4b}$$

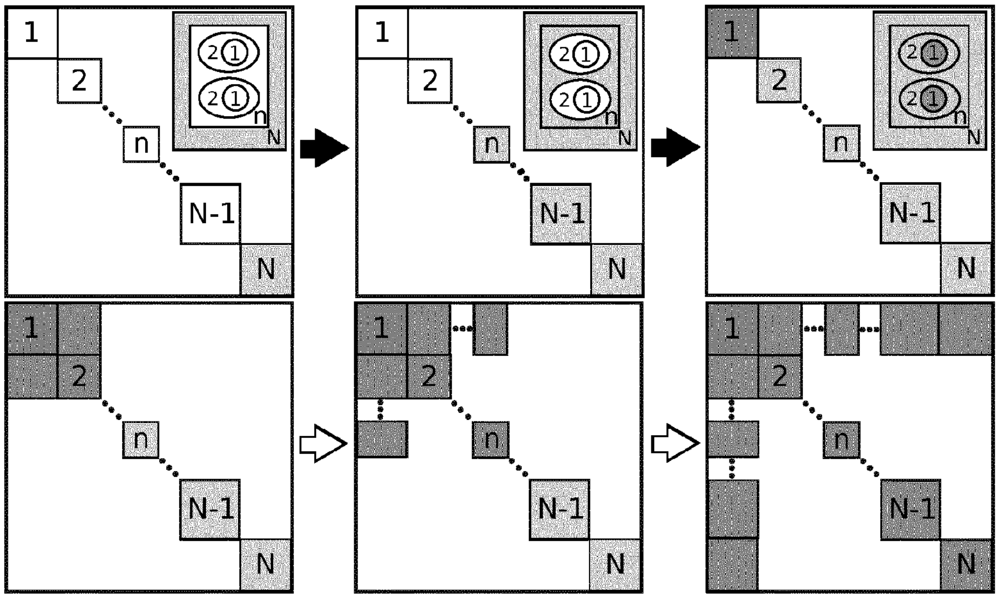


FIG. 10. Top row: recursive sweep going from cell $n = N$ to $n = 1$. Light gray indicate blocks that are stored for the reversed sweep and dark gray indicate blocks of the full GF. The inset shows an illustration of how the different blocks correspond to neighboring cells in the device region. Bottom row: reversed recursive sweep going from $n = 1$ 1 to $n = N$ showing how this sweep can obtain both diagonal and off-diagonal blocks.

Appendix B: Pseudomagnetic field for rotational symmetric strain field

The strain tensor is generally given as

$$\epsilon_{ij} = \frac{1}{2}\Big(\partial_j u_i + \partial_i u_j + (\partial_i z)(\partial_j z)\Big), \quad i,j = x,y, \quad \text{(B1)}$$

where ***u***($x$, $y$) is the in-plane deformation field and $z$($x$, $y$) is the out-of-plane deformation.[44]

A general two dimensional strain field, $\varepsilon_{ij}$ ($x$, $y$), leads to a gauge field in the effective Dirac Hamiltonian of graphene[43,44]

$$\boldsymbol{A} = -\frac{\hbar\beta}{2ea_0}\begin{pmatrix}\epsilon_{xx} - \epsilon_{yy} \\ -2\epsilon_{xy}\end{pmatrix}, \qquad \text{(B2)}$$

which in turn gives to the PMF

$$B_s = \boldsymbol{\nabla} \times \boldsymbol{A} = \partial_x A_y - \partial_y A_x. \qquad \text{(B3)}$$

Eqs. (B2) and (B3) implies that the x-axis is chosen along the zigzag direction of the graphene lattice.

Now restricting ourselves to rotational symmetric deformations, $u(r) = u_r$ and $z(r) = z$, while using polar coordinates ($r$, $\theta$) yields

$$B_s = -\frac{\hbar\beta}{2ea_0}\left(2\frac{g(r)}{r} - \partial_r g(r)\right)\sin(3\theta), \qquad \text{(B4)}$$

with $g(r) = \partial_r u_r - u_r/r + \frac{1}{2}(\partial_r z)^2$. We notice from Eq. (B4) that the PMF for a rotational symmetric displacement field is always 6-fold symmetric. On the other hand, the magnitude depends on both the in-plane and out-of-plane displacement.

Considering the displacement field in Eq. (13) we now obtain a PMF of the form,

$$B_s = -\frac{\hbar\beta u_0}{2ea_0R^2}\sin(3\theta). \qquad \text{(B5)}$$

Taking into account the scaling $u_0 \propto h_0^2/R$, we obtain a final scaling of the PMF with the size of the bubble, $B_s \propto h_0^2/R^3$.

* mikse@nanotech.dtu.dk

[1] A. K. Geim and I. V. Grigorieva, Nature 499, 419 (2013).

[2] G. Fiori, F. Bonaccorso, G. Iannaccone, T. Palacios, D. Neumaier, A. Seabaugh, S. K. Banerjee, and L. Colombo, Nature Nanotechnology 9, 768 (2014).

[3] J. Bai, X. Zhong, S. Jiang, Y. Huang, and X. Duan, Nature nanotechnology 5, 190 (2010).

[4] F. Schedin, A. K. Geim, S. V. Morozov, E. W. Hill, P. Blake, M. I. Katsnelson, and K. S. Novoselov, Nature Materials 6, 652 (2007).

[5] Z. Qi, D. A. Bahamon, V. M. Pereira, H. S. Park, D. K. Campbell, and A. H. C. Neto, Nano letters 13, 2692 (2013).

[6] K. S. Novoselov and A. H. Castro Neto, Physica Scripta T146, 014006 (2012).

[7] A. H. Castro Neto, N. M. R. Peres, K. S. Novoselov, and A. K. Geim, Reviews of Modern Physics 81, 109 (2009).

[8] C. Lewenkopf and E. Mucciolo, Journal of Computational Electronics 12, 203 (2013).

[9] S. Datta, Electronic Transport in Mesoscopic Systems (Cambridge University Press, 1997).

[10] L. E. F. F. Torres, S. Roche, and J.-C. Charlier, Introduction to Graphene-Based Nanomaterials (Cambridge University Press, 2014).

[11] K. Kazymyrenko and X. Waintal, Physical Review B 77, 115119 (2008).

[12] M. Wimmer and K. Richter, Journal of Computational Physics 228, 8548 (2009).

[13] J. Baringhaus, M. Ruan, F. Edler, A. Tejeda, M. Sicot, A.-P. Li, Z. Jiang, E. H. Conrad, C. Berger, C. Tegenkamp, and W. A. de Heer, Nature 506, 349 (2014).

[14] P. W. Sutter, J.-I. Flege, and E. A. Sutter, Nature Materials 7, 406 (2008).

[15] M. Settnes, S. R. Power, D. H. Petersen, and A.-P. Jauho, Phys. Rev. Lett. 112, 096801 (2014).

[16] S. R. Power and M. S. Ferreira, Physical Review B 83, 155432 (2011).

[17] C. Bena, Physical Review B 79, 125427 (2009).

[18] J. A. Lawlor and M. S. Ferreira, ArXiv e-prints (2014), arXiv:1411.6240.

[19] S. R. Power, P. D. Gorman, J. M. Duffy, and M. S. Ferreira, Phys. Rev. B 86, 195423 (2012).

[20] D. A. Areshkin and B. K. Nikolić, Physical Review B 81, 155450 (2010).

[21] M. Yang, X.-J. Ran, Y. Cui, and R.-Q. Wang, Chinese Physics B 20, 097201 (2011).

[22] G. Thorgilsson, G. Viktorsson, and S. Erlingsson, Journal of Computational Physics 261, 256 (2014).

[23] G. Metalidis and P. Bruno, Physical Review B 72, 235304 (2005).

[24] A. Cresti, R. Farchioni, G. Grosso, and G. Parravicini, Physical Review B 68, 075306 (2003).

[25] R. N. Sajjad, C. A. Polanco, and A. W. Ghosh, Journal of Computational Electronics 12, 232 (2013).

[26] E. Costa Girão and V. Meunier, Journal of Computational Electronics 12, 123 (2013).

[27] T. Low, F. Guinea, and M. I. Katsnelson, Physical Review B 83, 195436 (2011).

[28] F. Guinea, M. I. Katsnelson, and A. K. Geim, Nature Physics 6, 30 (2009).

[29] G. W. Jones and V. M. Pereira, New Journal of Physics 16, 093044 (2014).

[30] Z. Qi, A. L. Kitt, H. S. Park, V. M. Pereira, D. K. Campbell, and A. H. Castro Neto, Phys. Rev. B 90, 125419 (2014).

[31] M. Neek-Amal and F. M. Peeters, Phys. Rev. B 85, 195445 (2012).

[32] J. Lu, A. H. Castro Neto, and K. P. Loh, Nature communications 3, 823 (2012).

[33] R. Carrillo-Bastos, D. Faria, A. Latgé, F. Mireles, and N. Sandler, Physical Review B 90, 041411 (2014)

[34] F. D. Juan, A. Cortijo, M. A. H. Vozmediano, and A. Cano, Nature Physics 7, 810 (2011).

[35] M. Neek-Amal, L. Covaci, K. Shakouri, and F. M. Peeters, Physical Review B 88, 115428 (2013).

[36] M. Neek-Amal and F. M. Peeters, Physical Review B 85, 195446 (2012).

[37] V. Pereira, A. H. Castro Neto, and N. M. R. Peres, Physical Review B 80, 045401 (2009).

[38] V. M. Pereira and A. H. Castro Neto, Phys. Rev. Lett. 103, 046801 (2009).

[39] V. M. Pereira, R. M. Ribeiro, N. M. R. Peres, and A. H. Castro Neto, EPL (Europhysics Letters) 92, 67001 (2010).

[40] D. Moldovan, M. Ramezani Masir, and F. M. Peeters, Physical Review B 88, 035446 (2013).

[41] F. M. D. Pellegrino, G. G. N. Angilella, and R. Pucci, Physical Review B 81, 035411 (2010).

[42] Z. H. Ni, T. Yu, Y. H. Lu, Y. Y. Wang, Y. P. Feng, and Z. X. Shen, ACS Nano 2, 2301 (2008).

[43]H. Suzuura and T. Ando, Phys. Rev. B 65, 235412 (2002).

[44] M. Vozmediano, M. Katsnelson, and F. Guinea, Physics Reports 496, 109 (2010).

[45] N. Levy, S. a. Burke, K. L. Meaker, M. Panlasigui, a. Zettl, F. Guinea, A. H. Castro Neto, and M. F. Crommie, Science 329, 544 (2010).

[46] J. S. Bunch, S. S. Verbridge, J. S. Alden, A. M. van der Zande, J. M. Parpia, H. G. Craighead, and P. L. McEuen, Nano Letters 8, 2458 (2008).

[47] K. Yue, W. Gao, R. Huang, and K. M. Liechti, Journal of Applied Physics 112, 083512 (2012).

[48] F. M. D. Pellegrino, G. G. N. Angilella, and R. Pucci, Phys. Rev. B 84, 195404 (2011).

[49] T. G. Pedersen, C. Flindt, J. G. Pedersen, N. A. Mortensen, A.-P. Jauho, and K. Pedersen, Physical Review Letters 100, 136804 (2008).

[50] T. Gunst, T. Markussen, A.-P. Jauho, and M. Brandbyge, Phys. Rev. B 84, 155449 (2011).

[51] J. A. Fürst, J. G. Pedersen, C. Flindt, N. A. Mortensen, M. Brandbyge, T. G. Pedersen, and A. P. Jauho, New Journal of Physics 11, 095020 (2009).

[52] J. G. Pedersen, T. Gunst, T. Markussen, and T. G. Pedersen, Phys. Rev. B 86, 245410 (2012).

[53] S. R. Power and A.-P. Jauho, Phys. Rev. B 90, 115408 (2014).

[54] G. F. Schneider, S. W. Kowalczyk, V. E. Calado, G. Pandraud, H. W. Zandbergen, L. M. K. Vandersypen, and C. Dekker, Nano letters 10, 3163 (2010).

[55] C. A. Merchant, K. Healy, M. Wanunu, V. Ray, N. Peterman, J. Bartel, M. D. Fischbein, K. Venta, Z. Luo, A. T. C. Johnson, and M. Drndić, Nano letters 10, 2915 (2010).

[56] M. Settnes, S. R. Power, D. H. Petersen, and A.-P. Jauho, Phys. Rev. B 90, 035440 (2014).

[57] M. Kim, N. S. Safron, E. Han, M. S. Arnold, and P. Gopalan, Nano letters 10, 1125 (2010).

[58] M. Kim, N. S. Safron, E. Han, M. S. Arnold, and P. Gopalan, ACS Nano 6, 9846 (2012).

[59] J. Eroms and D. Weiss, New Journal of Physics 11, 095021 (2009).

[60] Q. Xu, M.-Y. Wu, G. F. Schneider, L. Houben, S. K. Malladi, C. Dekker, E. Yucelen, R. E. Dunin-Borkowski, and H. W. Zandbergen, ACS Nano 7, 1566 (2013).

[61] F. Oberhuber, S. Blien, S. Heydrich, F. Yaghobian, T. Korn, C. Schuller, C. Strunk, D. Weiss, and J. Eroms, Applied Physics Letters 103, 143111 (2013).

[62] X. Jia, M. Hofmann, V. Meunier, B. G. Sumpter, J. Campos-Delgado, J. M. Romo-Herrera, H. Son, Y.-P. Hsieh, A. Reina, J. Kong, M. Terrones, and M. S. Dresselhaus, Science 323, 1701 (2009).

[63] F. Pizzocchero, M. Vanin, J. Kling, T. W. Hansen, K. W. Jacobsen, P. Bøggild, and T. J. Booth, The Journal of Physical Chemistry C 118, 4296 (2014).

[64] A. Cagliani, D. Mackenzie, L. Tschammer, F. Pizzocchero, K. Almdal, and P. Bøggild, Nano Research 7, 743 (2014).

[65] J. Kling, J. S. Vestergaard, A. B. Dahl, N. Stenger, T. J. Booth, P. Bggild, R. Larsen, J. B. Wagner, and T. W. Hansen, Carbon 74, 363 (2014).

[66] J. S. Vestergaard, J. Kling, A. B. Dahl, T. W. Hansen, J. B. Wagner, and R. Larsen, Microscopy and Microanalysis, 1 (2014).

[67] K. W. Clark, X.-G. Zhang, G. Gu, J. Park, G. He, R. M. Feenstra, and A.-P. Li, Physical Review X 4, 011021 (2014).

[68] A.-P. Li, K. W. Clark, X.-G. Zhang, and A. P. Baddorf, Advanced Functional Materials 23, 2509 (2013)

[69] M. Settnes, S. R. Power, D. H. Petersen, and A.-P. Jauho, Phys. Rev. Lett. 112, 096801 (2014).

[70] E. N. Economou, Green's functions in quantum Physics (Springer, 2005).

[71] The surface Green's function of the single atomic chain is given by $g^s = \frac{E \pm \sqrt{E^2 - 4\gamma^2}}{2\gamma^2}$. We chose $\gamma = 1|\gamma_{cc}|$ as this gives a constant DOS within the considered energies. Adding a decay to the off-diagonal terms finally gives the GF, $g^s_{ij} = g^s(\delta_{ij} + \delta_{i \neq j}/r_{ij})$, where $r_{ij}$ is the distance between the sites *i* and *j*.

## Claims

**1.** A method for predicting the electronic properties of a two-dimensional system, the method comprising the steps of

1) considering the two-dimensional system as an infinite system without boundaries,
2) converting the infinite system to a two-dimensional system having one or more boundaries, and
3) applying an analytical approach to the two-dimensional system in order to predict the electronic properties of the two-dimensional system when said two-dimensional system is considered an infinite system.

**2.** A method according to claim 1, wherein the step of applying the analytical approach involves an appliance of Green's function methods.

**3.** A method according to claim 2, wherein the step of applying Green's function methods involve an appliance of a combination of analytical Green's function and recursive Green's function methods.

**4.** A method according to any of claims 1-3, wherein the two-dimensional system comprises a two-dimensional graphene system.

**5.** A method for predicting the electronic properties of a device comprising a two-dimensional system, the method comprising the steps of

1) considering the two-dimensional system as an infinite system without boundaries,
2) converting the infinite system to a two-dimensional system having one or more boundaries, and
3) applying an analytical approach to the two-dimensional system in order to predict the electronic properties of the two-dimensional system when said two-dimensional system is considered an infinite system.

**6.** A method according to claim 5, wherein the step of applying the analytical approach involves an appliance of Green's function methods.

**7.** A method according to claim 6, wherein the step of applying Green's function methods involve an appliance of a combination of analytical Green's function and recursive Green's function methods.

**8.** A method according to any of claims 5-7, wherein the two-dimensional system comprises a two-dimensional graphene system.

**9.** Use of the method according to any of claims 1-4 to predict the electronic properties of a device comprising a two-dimensional system.

**10.** Use according to claim 9, wherein the two-dimensional system comprises a two-dimensional graphene system.

Europäisches Patentamt
European Patent Office
Office européen des brevets

# EUROPEAN SEARCH REPORT

Application Number
EP 14 20 0624

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | LEWENKOPF C. H. ET AL: "The recursive Green's function method for graphene", JOURNAL OF COMPUTATIONAL ELECTRONICS, vol. 12, no. 2, 4 May 2013 (2013-05-04), pages 203-231, XP055197854, ISSN: 1569-8025, DOI: 10.1007/s10825-013-0458-7<br>* abstract *<br>* page 203, right-hand column, line 3 - page 204, left-hand column, line 9 *<br>* paragraph [0002] *<br>* paragraph [0003] *<br>* paragraph [04.1] *<br>* paragraph [0005] *<br>----- | 1-10 | INV.<br>G06F19/00 |
| X | FIORI G. ET AL: "Multiscale Modeling for Graphene-Based Nanoscale Transistors", PROCEEDINGS OF THE IEEE, IEEE. NEW YORK, US, vol. 101, no. 7, 1 July 2013 (2013-07-01), pages 1653-1669, XP011515034, ISSN: 0018-9219, DOI: 10.1109/JPROC.2013.2259451<br>* abstract *<br>* page 1654, right-hand column, lines 15-33 *<br>* paragraph [00B.] *<br>* paragraph [00D.] *<br>----- | 1-10 | TECHNICAL FIELDS SEARCHED (IPC)<br>G06F |

-/--

The present search report has been drawn up for all claims

1

| Place of search | Date of completion of the search | Examiner |
|---|---|---|
| The Hague | 25 June 2015 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 20 0624

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WIMMER M. ET AL: "Optimal block-tridiagonalization of matrices for coherent charge transport", JOURNAL OF COMPUTATIONAL PHYSICS, LONDON, GB, vol. 228, no. 23, 10 December 2009 (2009-12-10), pages 8548-8565, XP026684031, ISSN: 0021-9991, DOI: 10.1016/J.JCP.2009.08.001 [retrieved on 2009-08-12] * paragraph [001.] * * paragraph [003.] - paragraph [3.2.] * ----- | 1-10 | |
| X | SHOKRI A. A. ET AL: "Electronic transport of graphene nanoribbons within recursive Greens function", SUPERLATTICES AND MICROSTRUCTURES, ACADEMIC PRESS, LONDON, GB, vol. 51, no. 4, 27 January 2012 (2012-01-27), pages 523-532, XP028467963, ISSN: 0749-6036, DOI: 10.1016/J.SPMI.2012.01.016 [retrieved on 2012-02-06] * the whole document * ----- | 1-10 | TECHNICAL FIELDS SEARCHED (IPC) |
| A | LAWLOR J. A. ET AL: "Sublattice asymmetry of impurity doping in graphene: A review", BEILSTEIN JOURNAL OF NANOTECHNOLOGY, vol. 5, 5 August 2014 (2014-08-05), pages 1210-1217, XP055198037, DOI: 10.3762/bjnano.5.133 * figure 4 * ----- | 1-10 | |

-/--

The present search report has been drawn up for all claims

1

| Place of search | Date of completion of the search | Examiner |
|---|---|---|
| The Hague | 25 June 2015 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 20 0624

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | CASTRO NETO A. H. ET AL: "The electronic properties of graphene", REVIEWS OF MODERN PHYSICS, vol. 81, no. 1, 14 January 2009 (2009-01-14), pages 109-162, XP055198131, ISSN: 0034-6861, DOI: 10.1103/RevModPhys.81.109<br>* the whole document *<br>----- | | |

TECHNICAL FIELDS SEARCHED (IPC)

The present search report has been drawn up for all claims

1

| Place of search | Date of completion of the search | Examiner |
|---|---|---|
| The Hague | 25 June 2015 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **A. K. GEIM ; I. V. GRIGORIEVA.** *Nature,* 2013, vol. 499, 419
- **G. FIORI ; F. BONACCORSO ; G. IANNACCONE ; T. PALACIOS ; D. NEUMAIER ; A. SEABAUGH ; S. K. BANERJEE ; L. COLOMBO.** *Nature Nanotechnology,* 2014, vol. 9, 768
- **J. BAI ; X. ZHONG ; S. JIANG ; Y. HUANG ; X. DUAN.** *Nature nanotechnology,* 2010, vol. 5, 190
- **F. SCHEDIN ; A. K. GEIM ; S. V. MOROZOV ; E. W. HILL ; P. BLAKE ; M. I. KATSNELSON ; K. S. NOVOSELOV.** *Nature Materials,* 2007, vol. 6, 652
- **Z. QI ; D. A. BAHAMON ; V. M. PEREIRA ; H. S. PARK ; D. K. CAMPBELL ; A. H. C. NETO.** *Nano letters,* 2013, vol. 13, 2692
- **K. S. NOVOSELOV ; A. H. CASTRO NETO.** *Physica Scripta,* 2012, vol. T146, 014006
- **A. H. CASTRO NETO ; N. M. R. PERES ; K. S. NOVOSELOV ; A. K. GEIM.** *Reviews of Modern Physics,* 2009, vol. 81, 109
- **C. LEWENKOPF ; E. MUCCIOLO.** *Journal of Computational Electronics,* 2013, vol. 12, 203
- **S. DATTA.** Electronic Transport in Mesoscopic Systems. Cambridge University Press, 1997
- **L. E. F. F. TORRES ; S. ROCHE ; J.-C. CHARLIER.** Introduction to Graphene-Based Nanomaterials. Cambridge University Press, 2014
- **K. KAZYMYRENKO ; X. WAINTAL.** *Physical Review B,* 2008, vol. 77, 115119
- **M. WIMMER ; K. RICHTER.** *Journal of Computational Physics,* 2009, vol. 228, 8548
- **J. BARINGHAUS ; M. RUAN ; F. EDLER ; A. TEJEDA ; M. SICOT ; A.-P. LI ; Z. JIANG ; E. H. CONRAD ; C. BERGER ; C. TEGENKAMP.** *Nature,* 2014, vol. 506, 349
- **P. W. SUTTER ; J.-I. FLEGE ; E. A. SUTTER.** Nature Materials. 2008, vol. 7, 406
- **M. SETTNES ; S. R. POWER ; D. H. PETERSEN ; A.-P. JAUHO.** *Phys. Rev. Lett.,* 2014, vol. 112, 096801
- **S. R. POWER ; M. S. FERREIRA.** *Physical Review B,* 2011, vol. 83, 155432
- **C. BENA.** *Physical Review B,* 2009, vol. 79, 125427
- **J. A. LAWLOR ; M. S. FERREIRA.** *ArXiv e-prints,* 2014, vol. arXiv, 1411-6240
- **S. R. POWER ; P. D. GORMAN ; J. M. DUFFY ; M. S. FERREIRA.** *Phys. Rev. B,* 2012, vol. 86, 195423
- **D. A. ARESHKIN ; B. K. NIKOLIĆ.** *Physical Review B,* 2010, vol. 81, 155450
- **M. YANG ; X.-J. RAN ; Y. CUI ; R.-Q. WANG.** *Chinese Physics B,* 2011, vol. 20, 097201
- **G. THORGILSSON ; G. VIKTORSSON ; S. ERLINGSSON.** *Journal of Computational Physics,* 2014, vol. 261, 256
- **G. METALIDIS ; P. BRUNO.** *Physical Review B,* 2005, vol. 72, 235304
- **A. CRESTI ; R. FARCHIONI ; G. GROSSO ; G. PARRAVICINI.** *Physical Review B,* 2003, vol. 68, 075306
- **R. N. SAJJAD ; C. A. POLANCO ; A. W. GHOSH.** *Journal of Computational Electronics,* 2013, vol. 12, 232
- **E. COSTA GIRÃO ; V. MEUNIER.** *Journal of Computational Electronics,* 2013, vol. 12, 123
- **T. LOW ; F. GUINEA ; M. I. KATSNELSON.** *Physical Review B,* 2011, vol. 83, 195436
- **F. GUINEA ; M. I. KATSNELSON ; A. K. GEIM.** *Nature Physics,* 2009, vol. 6, 30
- **G. W. JONES ; V. M. PEREIRA.** *New Journal of Physics,* 2014, vol. 16, 093044
- **Z. QI ; A. L. KITT ; H. S. PARK ; V. M. PEREIRA ; D. K. CAMPBELL ; A. H. CASTRO NETO.** *Phys. Rev. B,* 2014, vol. 90, 125419
- **M. NEEK-AMAL ; F. M. PEETERS.** *Phys. Rev. B,* 2012, vol. 85, 195445
- **J. LU ; A. H. CASTRO NETO ; K. P. LOH.** *Nature communications,* 2012, vol. 3, 823
- **R. CARRILLO-BASTOS ; D. FARIA ; A. LATGÉ ; F. MIRELES ; N. SANDLER.** *Physical Review B,* 2014, vol. 90, 041411
- **F. D. JUAN ; A. CORTIJO ; M. A. H. VOZMEDIANO ; A. CANO.** *Nature Physics,* 2011, vol. 7, 810
- **M. NEEK-AMAL ; L. COVACI ; K. SHAKOURI ; F. M. PEETERS.** *Physical Review B,* 2013, vol. 88, 115428
- **M. NEEK-AMAL ; F. M. PEETERS.** *Physical Review B,* 2012, vol. 85, 195446
- **V. PEREIRA ; A. H. CASTRO NETO ; N. M. R. PERES.** *Physical Review B,* 2009, vol. 80, 045401
- **V. M. PEREIRA ; A. H. CASTRO NETO.** *Phys. Rev. Lett.,* 2009, vol. 103, 046801
- **V. M. PEREIRA ; R. M. RIBEIRO ; N. M. R. PERES ; A. H. CASTRO NETO.** *EPL,* 2010, vol. 92, 67001

- **D. MOLDOVAN ; M. RAMEZANI MASIR ; F. M. PEETERS.** *Physical Review B,* 2013, vol. 88, 035446
- **F. M. D. PELLEGRINO ; G. G. N. ANGILELLA ; R. PUCCI.** *Physical Review B,* 2010, vol. 81, 035411
- **Z. H. NI ; T. YU ; Y. H. LU ; Y. Y. WANG ; Y. P. FENG ; Z. X. SHEN.** *ACS Nano,* 2008, vol. 2, 2301
- **H. SUZUURA ; T. ANDO.** *Phys. Rev. B,* 2002, vol. 65, 235412
- **M. VOZMEDIANO ; M. KATSNELSON ; F. GUINEA.** *Physics Reports,* 2010, vol. 496, 109
- **N. LEVY ; S. A. BURKE ; K. L. MEAKER ; M. PANLASIGUI ; A. ZETTL ; F. GUINEA ; A. H. CASTRO NETO ; M. F. CROMMIE.** *Science,* 2010, vol. 329, 544
- **J. S. BUNCH ; S. S. VERBRIDGE ; J. S. ALDEN ; A. M. VAN DER ZANDE ; J. M. PARPIA ; H. G. CRAIGHEAD ; P. L. MCEUEN.** *Nano Letters,* 2008, vol. 8, 2458
- **K. YUE ; W. GAO ; R. HUANG ; K. M. LIECHTI.** *Journal of Applied Physics,* 2012, vol. 112, 083512
- **F. M. D. PELLEGRINO ; G. G. N. ANGILELLA ; R. PUCCI.** *Phys. Rev. B,* 2011, vol. 84, 195404
- **T. G. PEDERSEN ; C. FLINDT ; J. G. PEDERSEN ; N. A. MORTENSEN ; A.-P. JAUHO ; K. PEDERSEN.** *Physical Review Letters,* 2008, vol. 100, 136804
- **T. GUNST ; T. MARKUSSEN ; A.-P. JAUHO ; M. BRANDBYGE.** *Phys. Rev. B,* 2011, vol. 84, 155449
- **J. A. FÜRST ; J. G. PEDERSEN ; C. FLINDT ; N. A. MORTENSEN ; M. BRANDBYGE ; T. G. PEDERSEN ; A. P. JAUHO.** *New Journal of Physics,* 2009, vol. 11, 095020
- **J. G. PEDERSEN ; T. GUNST ; T. MARKUSSEN ; T. G. PEDERSEN.** *Phys. Rev. B,* 2012, vol. 86, 245410
- **S. R. POWER ; A.-P. JAUHO.** *Phys. Rev. B,* 2014, vol. 90, 115408
- **G. F. SCHNEIDER ; S. W. KOWALCZYK ; V. E. CALADO ; G. PANDRAUD ; H. W. ZANDBERGEN ; L. M. K. VANDERSYPEN ; C. DEKKER.** *Nano letters,* 2010, vol. 10, 3163
- **C. A. MERCHANT ; K. HEALY ; M. WANUNU ; V. RAY ; N. PETERMAN ; J. BARTEL ; M. D. FISCHBEIN ; K. VENTA ; Z. LUO ; A. T. C. JOHNSON.** *Nano letters,* 2010, vol. 10, 2915
- **M. SETTNES ; S. R. POWER ; D. H. PETERSEN ; A.-P. JAUHO.** *Phys. Rev. B,* 2014, vol. 90, 035440
- **M. KIM ; N. S. SAFRON ; E. HAN ; M. S. ARNOLD ; P. GOPALAN.** *Nano letters,* 2010, vol. 10, 1125
- **M. KIM ; N. S. SAFRON ; E. HAN ; M. S. ARNOLD ; P. GOPALAN.** *ACS Nano,* 2012, vol. 6, 9846
- **J. EROMS ; D. WEISS.** *New Journal of Physics,* 2009, vol. 11, 095021
- **Q. XU ; M.-Y. WU ; G. F. SCHNEIDER ; L. HOUBEN ; S. K. MALLADI ; C. DEKKER ; E. YUCELEN ; R. E. DUNIN-BORKOWSKI ; H. W. ZANDBERGEN.** *ACS Nano,* 2013, vol. 7, 1566
- **F. OBERHUBER ; S. BLIEN ; S. HEYDRICH ; F. YAGHOBIAN ; T. KORN ; C. SCHULLER ; C. STRUNK ; D. WEISS ; J. EROMS.** *Applied Physics Letters,* 2013, vol. 103, 143111
- **X. JIA ; M. HOFMANN ; V. MEUNIER ; B. G. SUMPTER ; J. CAMPOS-DELGADO ; J. M. ROMO-HERRERA ; H. SON ; Y.-P. HSIEH ; A. REINA ; J. KONG.** *Science,* 2009, vol. 323, 1701
- **F. PIZZOCCHERO ; M. VANIN ; J. KLING ; T. W. HANSEN ; K. W. JACOBSEN ; P. BØGGILD ; T. J. BOOTH.** *The Journal of Physical Chemistry C,* 2014, vol. 118, 4296
- **A. CAGLIANI ; D. MACKENZIE ; L. TSCHAMMER ; F. PIZZOCCHERO ; K. ALMDAL ; P. BØGGILD.** *Nano Research,* 2014, vol. 7, 743
- **J. KLING ; J. S. VESTERGAARD ; A. B. DAHL ; N. STENGER ; T. J. BOOTH ; P. BGGILD ; R. LARSEN ; J. B. WAGNER ; T. W. HANSEN.** *Carbon,* 2014, vol. 74, 363
- **J. S. VESTERGAARD ; J. KLING ; A. B. DAHL ; T. W. HANSEN ; J. B. WAGNER ; R. LARSEN.** *Microscopy and Microanalysis,* 2014, 1
- **K. W. CLARK ; X.-G. ZHANG ; G. GU ; J. PARK ; G. HE ; R. M. FEENSTRA ; A.-P. LI.** *Physical Review X,* 2014, vol. 4, 011021
- **A.-P. LI ; K. W. CLARK ; X.-G. ZHANG ; A. P. BADDORF.** *Advanced Functional Materials,* 2013, vol. 23, 2509
- **E. N. ECONOMOU.** Green's functions in quantum Physics. Springer, 2005